(19) Europäisches Patentamt
European Patent Office
Office européen des brevets

(11) EP 0 691 843 B1

(12) **EUROPÄISCHE PATENTSCHRIFT**

(45) Veröffentlichungstag und Bekanntmachung des Hinweises auf die Patenterteilung:
**19.02.1997 Patentblatt 1997/08**

(21) Anmeldenummer: **94911955.6**

(22) Anmeldetag: **24.03.1994**

(51) Int. Cl.$^6$: **A61K 31/135**, A61K 9/20, A61K 9/48

(86) Internationale Anmeldenummer:
**PCT/EP94/00949**

(87) Internationale Veröffentlichungsnummer:
**WO 94/22434 (13.10.1994 Gazette 1994/23)**

(54) **RETARDMIKROTABLETTE VON BETA-PHENYLPROPIOPHENONDERIVATEN**
RETARDED-ACTION MICROTABLET MADE OF BETA-PHENYLPROPIOPHENONE DERIVATIVES
MICRO-COMPRIME RETARD DE DERIVES DE BETA-PHENYLPROPIOPHENONE

(84) Benannte Vertragsstaaten:
**AT BE CH DE DK ES FR GB GR IE IT LI LU NL PT SE**

(30) Priorität: **03.04.1993 DE 4310963**

(43) Veröffentlichungstag der Anmeldung:
**17.01.1996 Patentblatt 1996/03**

(73) Patentinhaber: **KNOLL AKTIENGESELLSCHAFT**
**D-67061 Ludwigshafen (DE)**

(72) Erfinder:
- **KOLTER, Karl**
**D-67117 Limburgerhof (DE)**
- **FRICKE, Helmut**
**D-67112 Mutterstadt (DE)**
- **BÜHLER, Volker**
**D-76135 Karlsruhe (DE)**
- **MÜLLER-PELTZER, Herbert**
**D-69120 Heidelberg (DE)**

(74) Vertreter: **Karau, Wolfgang, Dr.**
**BASF Aktiengesellschaft,**
**Patentabteilung ZDX - C 6**
**67056 Ludwigshafen (DE)**

(56) Entgegenhaltungen:
**EP-A- 0 334 167**
**WO-A-90/11755**
**WO-A-92/04013**

EP 0 691 843 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.15/3.4

## Beschreibung

Die Erfindung betrifft zylindrische Mikrotabletten von β-Phenylpropiophenonderivaten mit hohem Wirkstoffgehalt, hoher Wirkstoffdichte und retardierter und pressdruckunabhängiger Freisetzung ohne retardierende Hilfsstoffe.

Wenn oben und im folgenden von β-Phenylpropiophenonderivaten die Rede ist, so sind stets auch und besonders deren physiologisch unbedenklichen Salze, vorzugsweise das Hydrochlorid, gemeint.

Gemäß dem Stand der Technik wird die Wirkstofffreigabe von Tabletten entweder durch eine retardierende Matrix, in die der Wirkstoff eingebettet ist, oder durch einen retardierenden Überzug, durch den Verdauungsflüssigkeit ein- und der Wirkstoff ausdiffundiert, verzögert.

Beide Prinzipien haben erhebliche Nachteile. Z.B. enthalten Matrixtabletten relativ große Mengen Hilfsstoffe, so daß das Tablettenvolumen bei gegebener Wirkstoffdosis relativ groß ist, was für den Patient unangenehm ist. Andererseits sind Filmtabletten aufwendig in der Herstellung und vor allem mechanisch empfindlich. Bei geringster Beschädigung des Lackfilms besteht die Gefahr einer plötzlichen Freisetzung des gesamten Wirkstoffgehaltes ("dose dumping"), was höchst unerwünscht ist (örtliche und zeitliche Überdosis mit schädlichen Nebenwirkungen; kurze Gesamtwirkzeit).

Sowohl Matrix- wie Film-Retardtabletten haben normalerweise Durchmesser von etwa 6 bis 12 mm und mehr und können daher den geschlossenen Pylorus nicht passieren. Die Freisetzung und Resorption ihres gesamten, auf eine Stelle im Magen-Darm-Trakt konzentrierten Wirkstoffgehaltes ist von den an dieser Stelle herrschenden Bedingungen abhängig, was eine starke interindividuelle und intraindividuelle Streuung des Plasmaspiegels zur Folge hat.

Diese Streuung ist bei den "Multiple-Unit"-Retardformen kleiner, da sich die "Units" gleichmäßig über den Magen- und Darmtrakt verteilen und auch den geschlossenen Pylorus passieren können. Als Multiple-Unit-Formen kommen in der Regel Pellets mit einem Diffusionslack, abgefüllt in Gelatine-Steckkapseln, zum Einsatz. Die Herstellung von Matrix-Pellets ist nur bei sehr niedrig dosierten Arzneistoffen möglich, da aufgrund der großen Oberfläche noch mehr Matrixsubstanz als bei der Bolus-Retardtablette benötigt würde.

Beispielsweise aus den Patentanmeldungen GB 2 176 999 und WO 92/04013 sind kleine Matrix-Retardtabletten bekannt, die ebenfalls größere Mengen an Retardierungshilfsstoffen enthalten. In der Patentanmeldung EP 22 17 32 werden Retardtabletten von schwerlöslichen Wirkstoffen beansprucht, die 60 bis 80 % Wirkstoff neben mindestens vier Hilfsmitteln enthalten. Diese Bolus-Formen sind, wie im Patent beschrieben, in ihrer Freisetzung stark abhängig vom Granulationsverfahren und dem bei der Herstellung verwendeten Gerät.

Weiterhin ist allgemein bekannt, daß bei der Tablettenherstellung mit einer Steigerung der Preßkraft eine Verlangsamung der Wirkstofffreisetzung einhergeht. Dies gilt sowohl für schnell freisetzende Tabletten als auch für Retardtabletten (Patentanmeldung WO 92/00064). Da trotz modernster Maschinentechnik die Preßkräfte schwanken, resultieren daraus unterschiedliche Freisetzungsgeschwindigkeiten. Hinzu kommen ferner noch von Charge zu Charge unterschiedliche Preßeigenschaften, die auf der Variabilität des zu verpressenden Granulates beruhen. Unterschiede in der Korngröße, Porosität, Oberflächenstruktur, Benetzbarkeit usw. können großen Einfluß auf die Preßeigenschaften und die Retardierung haben.

Der Erfindung lag die Aufgabe zugrunde, die Nachteile des Standes der Technik zu überwinden, d.h. Propafenon- und Diprafenon-Tabletten von geringer Größe, hohem Wirkstoffgehalt, hoher Wirkstoffdichte und preßdruckunabhängiger und über einen längeren Zeitraum gleichmäßiger Wirkstofffreigabe zu entwickeln.

Die Lösung dieser Aufgabe besteht in den Mikrotabletten nach den Ansprüchen 1 bis 4. Es wurde nämlich überraschend gefunden, daß im vorliegenden Fall die Herstellung von Retardtabletten ohne retardierende Hilfsstoffe möglich ist. Dies ist umso überraschender, als andere Arzneistoffe mit ähnlicher Wasserlöslichkeit wie Propafenon-Hydrochlorid (0,7 %), beispielsweise Cimetidin-Hydrochlorid oder Paracetamol, bei gleicher Präparation bereits in 1 Stunde zu 90 % freigesetzt werden.

Bei Propafenon-HCl handelt es sich um eine im Vergleich zu anderen außerordentlich schlecht preßbare Substanz. Eine Bolustablette mit handelsüblichen Dosierungen von 150 bis 300 mg und einem Wirkstoffgehalt über 80 % ist unter Produktionsbedingungen nicht herstellbar. Hingegen können die erfindungsgemäßen Mikrotabletten überraschenderweise mit verhältnismäßig hoher Maschinengeschwindigkeit ohne Probleme hinsichtlich Abrieb- und Bruchfestigkeit hergestellt werden, und zwar mit Wirkstoffgehalten im Bereich von 81 bis 99,9, vorzugsweise 85 bis 99,5 Gew.-% und einer Wirkstoffdichte von über 1 g/cm$^3$. So hohe Gehalte an derartigen Wirkstoffen in Tabletten sind bisher unerreicht.

Die erfindungsgemäßen Mikrotabletten sind zylindrisch mit flacher oder konvexer Ober- und Unterseite und mit unabhängig voneinander 1 bis 3, vorzugsweise 1,5 bis 2,5 mm Durchmesser und Höhe, wobei beide vorzugsweise etwa gleich groß sind.

Ferner war nicht vorherzusehen, daß die Wirkstofffreisetzung im Gegensatz zu den üblichen Erfahrungen vom Druck beim Pressen der Tabletten und auch über einen großen Bereich vom pH des Milieus praktisch unabhängig ist. "Praktisch unabhängig" heißt, daß der Einfluß für praktische Zwecke vernachlässigt werden kann. Die Konstanz der Freisetzung ist damit gewährleistet. Sie wird über die Größe der Tablette sowie gegebenenfalls durch die Freisetzung beschleunigende Zusätze so eingestellt, daß nach 3, vorzugsweise 5 Stunden höchstens 80, nach 24, vorzugsweise 15 Stunden mindestens 80 % des Wirkstoffs freigesetzt sind. Überraschenderweise weisen die erfindungsgemäßen

Mikrotabletten gegenüber herkömmlichen Retardformen, wie einer Bolusretardform mit ähnlicher In-vitro-Freisetzung, auch in vivo deutliche Vorzüge auf. Trotz kurzer Halbwertzeit bildet sich ein ausgeprägtes Blutspiegelplateau aus (Fig. 11). Die Fluktuation des Blutspiegels ist bei den Mikrotabletten erheblich geringer. Dies wird erkennbar an dem $t_{75\%}$-Wert (Zeitdauer im Dosierungsintervall, während der die Plasmaspiegel mindestens 75 % des maximalen Wertes betragen), der bei den erfindungsgemäßen Mikrotabletten bei 8 bis 9 Stunden liegt, gegenüber 5 bis 6 Stunden bei der Bolusretardform, sowie am PTF-Wert (peak to trough fluctuation; vgl. H. P. Koch und W. A. Ritschel, Synopsis der Biopharmazie und Pharmakokinetik, Ecomed-Verlagsgesellschaft mbH, Landsberg und München, 1986)

$$PTF\ (\%) = \frac{C_{max} - C_{min}}{\frac{AUC}{\Delta t}} \times 100$$

zum AUC-Wert vgl. J.K. Aronson et al., Europ. J. of Clinical Pharmacology Bd. 35 (1988), S. 1 bis 7.

der bei den Mikrotabletten nur etwa halb so groß wie bei den Bolusformen ist, und zwar kleiner als 75, vorzugsweise kleiner als 60 %. Die Mikrotabletten erbringen demnach eine erhöhte Therapiesicherheit, da überhöhte Plasmaspiegelspitzen und dadurch bedingte Nebenwirkungen nicht auftreten, der minimale, effektive Plasmaspiegel nicht unterschritten wird und diese Form in ihrer Bioverfügbarkeit durch Nahrungsaufnahme, im Gegensatz zur Bolusretardform, nicht beeinflußt wird.

Bei der Bolusretardform wird nüchtern ein um 50 % höherer AUC-Wert gefunden.

Generell zeigen die Mikrotabletten im Vergleich zur Bolusretardform geringere intra- und interindividuelle Unterschiede.

Die erfindungsgemäßen Mikrotabletten weisen ferner den Vorzug auf, daß sie, eingebracht in Magen- oder Darmsaft, keine Klebe- oder Anhaftungstendenzen aufweisen. Dadurch wird gewährleistet, daß sie als einzelne Formlinge den Magen- und Darmtrakt passieren und sich auch nicht an der Magen- oder Darmwand festsetzen und Irritationen auslösen. Solche Klebe- oder Anhaftungseigenschaften weisen beispielsweise kleine Formlinge mit hydrophilen Retardierungspolymeren auf (vgl. WO 92/04013).

Die Herstellung von Retardformen mit hydrophilen Retardierungspolymeren erfordert oft bei der Granulation den Einsatz von organischen Lösungsmitteln, damit nicht schon bei diesem Prozeßschritt Quellung einsetzt. Bei der Herstellung der erfindungsgemäßen Mikrotabletten kann darauf vollständig verzichtet werden.

Darreichungsformen mit hydrophilen Retardierungspolymeren weisen zudem den Nachteil auf, daß sie aufgrund der Sorptions- und Quellungstendenz empfindlich gegen eine Veränderung der Luftfeuchte bei Lagerung sind. Insbesondere hohe Luftfeuchten schaden diesen Zubereitungen. Die erfindungsgemäßen Mikrotabletten sind aufgrund der Unempfindlichkeit der Einsatzstoffe auch bei höheren Luftfeuchten stabil. Selbst nach 21tägiger Lagerung bei 93 % rel. Luftfeuchte liegt die Wasseraufnahme unter 1 %, und optisch ist keine Veränderung festzustellen.

Die Herstellung der erfindungsgemäßen Mikrotabletten erfolgt in pharmazeutisch üblichen Geräten und umfaßt folgende Schritte: Granulieren, Trocknen, Mischen, Tablettieren.

Die Korngröße des Wirkstoffs spielt im pharmazeutisch üblichen Rahmen bei der Herstellung der erfindungsgemäßen Mikrotabletten, entgegen allen Erwartungen, keine oder nur eine geringe Rolle. Dadurch ist es möglich, Propafenon-Hydrochlorid und Diprafenon-Hydrochlorid unterschiedlicher Korngröße zu Produkten gleicher Qualität zu verarbeiten.

Granulierung und Trocknung werden vorzugsweise im Wirbelbett durchgeführt. Die Agglomeration kann aber auch in einem horizontalen oder vertikalen Mischer durchgeführt werden.

Nach dem Durchgeben durch ein Sieb geeigneter Maschenweite wird das feuchte Granulat entweder im Umluft-Trockenschrank oder im Wirbelbett getrocknet. Die Korngröße des Granulates sollte unter 1 mm, bevorzugt unter 0,8 mm liegen.

Für die Agglomeration können alle gängigen Binde- oder Klebemittel eingesetzt werden, z.B. Polyvinylpyrrolidon, Vinylpyrrolidon-Vinylacetat-Copolymere, Gelatine, Hydroxypropylmethylzellulose, Hydroxypropylzellulose, Polymerisate aus Methacrylsäure und deren Estern. Durch die Verwendung einer Wirkstoff-Lösung als Granulationsflüssigkeit kann der Einsatz eines Bindemittels entfallen. Als Granulationsflüssigkeit wird Wasser ohne Zusätze bevorzugt.

Nach der Trocknung des Granulates auf den definierten Wassergehalt werden 0,1 bis 5, vorzugsweise 0,3 bis 2 Gew.-% eines Gleitmittels für die Tablettierung homogen untergemischt. Hierfür können ebenfalls alle gängigen Stoffe zum Einsatz kommen, wie Talkum, Magnesiumstearat, Calciumstearat, Stearinsäure, Calciumbehenat, Glycerinpalmitostearat, Natriumacetat, Polyethylenglykol, Natriumstearatfumarat. Außerdem können bis zu 18,9 Gew.-% weiterer üblicher Hilfsstoffe zugemischt werden, beispielsweise Farbstoffe, Stabilisatoren, Füllstoffe, Hydrophilisatoren, Fließregulierungsmittel, jedoch keine Retadierungsmittel.

Die Tablettierung erfolgt auf einer geeigneten, mit Mehrfach-Mikrotabletten-Stempeln bestückten Tablettenpresse. Die resultierenden Mikrotabletten besitzen eine zylindrische Form mit planer oder konvexer Oberfläche. Die Höhe und der Durchmesser können unabhängig voneinander variiert werden. Aus Gründen der höheren Schüttdichte und der

besseren Rieselfähigkeit ist es oft zweckmäßig, die Höhe der Mikrotabletten dem Durchmesser anzupassen.

Neben der Größe der Mikrotabletten liegt ein weiteres Steuerungselement der Freisetzung im Zusatz von Hydrophilisatoren, die die Lösungsgeschwindigkeit erhöhen. Als Hydrophilisatoren können einerseits Tenside, wie Polyoxyethylenfettsäureester, Polyoxyethylenfettalkoholether, Fettsäuresalze, Gallensäuresalze, Alkylsulfate oder Ethylenoxid-Propylenoxid-Blockpolymere, oder andererseits echt wasserlösliche Substanzen wie Polyethylenglykole, Harnstoff, Natriumchlorid, Sorbit, Mannit, Glycin, Nikotinamid, Zitronensäure-, Weinsäure- oder Phosphorsäuresalze verwendet werden. Die Freisetzungsrate nimmt dabei parallel zum Anstieg der Hydrophilisatorkonzentration zu.

Der Hydrophilisator kann bereits in das Granulat eingearbeitet oder aber erst zusammen mit dem Gleitmittel untergemischt werden. Dies ist natürlich nur bei festen Hydrophilisatoren möglich. Die Hydrophilisatorkonzentration beträgt 0,1 bis 15, in der Regel 1 bis 10 % der Gesamtmasse.

Zur Beschleunigung der Erosion des Wirkstoffs von der Tablettenoberfläche und damit der Wirkstofffreisetzung können auch Sprengmittel in Konzentrationen von 0,001 bis 0,5, vorzugsweise 0,01 bis 0,1 % verwendet werden, die weit unter den üblichen Konzentrationen liegen.

In der Regel können die Mikrotabletten direkt mit üblichen Füllmaschinen in Gelatinekapseln abgefüllt werden. Mitunter kann es von Vorteil sein, die Mikrotabletten vor der Abfüllung mit einem leicht löslichen, die Freisetzung nicht beeinflussenden Lackfilm zu versehen.

Außerdem ist es in vielen Fällen zweckmäßig, retardierte mit rasch freisetzenden oder weniger stark retardierten Mikrotabletten zu kombinieren. Dadurch wird zunächst eine Initialdosis freigesetzt, der sich die langsame Freisetzung der Erhaltungsdosis anschließt. Moderne Kapselfüllmaschinen sind in der Lage, zwei Produkte in eine Kapsel problemlos zu dosieren.

Die rasch freisetzende (Instant-Release-)Mikrotablette unterscheidet sich von der Retard-Mikrotablette dadurch, daß sie übliche Mengen an Sprengmittel, Quellmittel, Porenbildner enthält, die einen raschen Zerfall der Mikrotablette in kleine Bruchstücke und eine rasche Auflösung des Wirkstoffs bewirken.

Die Mikrotabletten der Beispiele hatten stets je 2 mm Durchmesser und Höhe, und die Wirkstoffdichte lag stets über 1.

## Beispiele

### Beispiel 1 (Fig. 1)

Propafenon-Retardmikrotabletten

Zusammensetzung

| | |
|---|---|
| Propafenon-HCl | 6,25 mg (96 %) |
| Hydroxypropylmethylcellulose | 0,20 mg |
| Magnesiumstearat | 0,05 mg |
| Gesamtgewicht | 6,50 mg |

In einem Wirbelschichtgranulator wurden 30 kg Propafenon-HCl mit 10 kg einer 10 %igen Hydroxypropylmethylcellulose-Lösung (Pharmacoat® 603) granuliert und getrocknet. Nach dem Durchgeben durch ein Sieb geeigneter Maschenweite wurde das Granulat in einem Pflugscharmischer mit der vorgegebenen Menge Magnesiumstearat gemischt.

Die Verpressung zu Mikrotabletten erfolgte auf einer mit Mehrfach-Mikrotabletten-Stempeln ausgerüsteten Rundläufer-Tablettenpresse.

Die der zu verabreichenden Dosis entsprechende Anzahl Mikrotabletten wurde mit einer geeigneten Kapselfüllmaschine in Hartgelatinekapseln abgefüllt.

Tabelle 1

| Ergebnisse von Probandenstudien mit Propafenon-HCl-Mikrotabletten von Beispiel 1 und einer Bolusretardform gemäß dem Vergleichsversuch (n = 18, Dosis: 400 mg Propafenon-HCl, repetierte Gabe) | | | | |
|---|---|---|---|---|
| | Mikrotabletten | | Bolusretardform | |
| | nüchtern | mit Nahrung | nüchtern | mit Nahrung |
| AUC $\frac{ng \cdot h}{ml}$ | 5 500 | 5 500 | 6 900 | 4 700 |
| $t_{75\,\%}$ (h) | 8-9 | 8-9 | 5-6 | 5-6 |
| PTF (%) | 52 | 56 | 88 | 106 |
| n = Zahl der Probanden<br>ng = Nanogramm<br>h = Stunden | | | | |

Beispiel 2 (Fig. 2)

Propafenon-Retardmikrotabletten

Zusammensetzung

| | |
|---|---|
| Propafenon-HCl | 5,92 mg (91 %) |
| Hydroxypropylmethylcellulose | 0,20 mg |
| Poloxamer 188 (USP) | 0,33 mg |
| Magnesiumstearat | 0,05 mg |
| Gesamtgewicht | 6,5 mg |

Die Herstellung erfolgte analog Beispiel 1. Die erforderliche Poloxamer 188-Menge wurde zusammen mit dem Magnesiumstearat in einem Pflugscharmischer unter das Granulat gemischt.

Beispiel 3 (Fig. 3)

Propafenon-Retardmikrotabletten

Zusammensetzung

| | |
|---|---|
| Propafenon-HCl | 5,61 mg (86 %) |
| Hydroxypropylmethylcellulose | 0,19 mg |
| Poloxamer 188 | 0,65 mg |
| Magnesiumstearat | 0,05 mg |
| Gesamtgewicht | 6,5 mg |

Die Herstellung erfolgte analog Beispiel 2.

Beispiel 4 (Fig. 4)

Propafenon-Retardmikrotabletten

Zusammensetzung

| | |
|---|---|
| Propafenon-HCl | 6,0 mg (86 %) |
| Hydroxypropylmethylcellulose | 0,2 mg |
| Calciumhydrogenphosphat | 0,613 mg |
| Monoglyceride (Myvatox®) | 0,15 mg |
| Vernetztes Polyvinylpyrrolidon | 0,007 mg |
| Magnesiumstearat | 0,03 mg |
| Gesamtgewicht | 7,0 mg |

Die Herstellung erfolgte analog Beispiel 2.

Beispiel 5 (Fig. 5)

Propafenon-Retardmikrotabletten

Zusammensetzung

| | |
|---|---|
| Propafenon-HCl | 5,70 mg (81 %) |
| Gelatine | 0,18 mg |
| Calciumhydrogenphosphat | 0,38 mg |
| NaCl | 0,70 mg |
| Magnesiumstearat | 0,04 mg |
| Gesamtgewicht | 7,0 mg |

Die Herstellung erfolgte analog Beispiel 1. Als Granulationsmittel wurde eine 10 %ige Gelatinelösung verwendet. Die NaCl-Menge wurde mit dem Magnesiumstearat untergemischt.

Beispiel 6 (Fig. 6)

Propafenon-Retardmikrotabletten

Zusammensetzung

| | |
|---|---|
| Propafenon-HCl | 5,83 mg (83 %) |
| Hydroxypropylmethylcellulose | 0,17 mg |
| β-Cyclodextrin | 0,9 mg |
| Magnesiumstearat | 0,1 mg |
| Gesamtgewicht | 7,0 mg |

Die Herstellung erfolgte analog Beispiel 2.

Beispiel 7 (Fig. 7)

Gelatinekapseln mit Propafenon-Retardmikrotabletten und Propafenon Instant-Release-Mikrotabletten

Zur Erzielung einer höheren initialen Freisetzung wurden auf einer geeigneten Kapselfüllmaschine 14 Instant-Release-Mikrotabletten und 55 Retard-Mikrotabletten in Hartgelatinekapseln gefüllt.

Zusammensetzung der Instant-Release-Mikrotabletten

| | |
|---|---|
| Propafenon-HCl | 6,05 mg (93 %) |
| Hydroxypropylmethylcellulose | 0,20 mg |
| Natriumcarboxymethylstärke | 0,20 mg |
| Magnesiumstearat | 0,05 mg |
| Gesamtgewicht | 6,5 mg |

Die Herstellung der Instant-Release-Mikrotabletten erfolgte analog Beispiel 2. Die Herstellung der eingesetzten Retard-Mikrotabletten erfolgte nach Beispiel 1.

Beispiel 8 (Fig. 8)

Propafenon-Retardmikrotabletten

Zusammensetzung

| | |
|---|---|
| Propafenon-HCl | 6,48 mg (99,7 %) |
| Magnesiumstearat | 0,02 mg |
| Gesamtgewicht | 6,50 mg |

Propafenon-Hydrochlorid und Magnesiumstearat wurden in einem Pflugscharmischer gemischt und anschließend zu Mikrotabletten verpreßt.

Die Bestimmung der In-vitro-Freisetzungskurven (Fig. 1 bis 10) erfolgte mit einer Paddle-Apparatur nach USP, wobei in den ersten beiden Stunden 0,08 molare HCl und anschließend Phosphatpuffer pH 6,8 verwendet wurde. Die Umdrehungsgeschwindigkeit lag bei 50 rpm.

Vergleichsversuch

Propafenon-Retardbolusfilmtablette

Zusammensetzung

| | |
|---|---|
| Propafenon-HCl | 450,0 mg |
| Natriumalginat | 112,0 mg |
| Mikrokristalline Cellulose Typ PH 101 | 37,0 mg |
| Copolymerisate aus Acryl- und Methacrylsäureestern mit einem geringen Gehalt an quartären Ammoniumgruppen (Eudragit® RS) | 15,0 mg |
| Gelatine | 55,0 mg |
| Magnesiumstearat | 3,5 mg |
| Mikrokristalline Cellulose Typ PH 102 | 12,5 mg |
| leicht löslicher Filmüberzug | 15,0 mg |
| Gesamtgewicht | 700,0 mg |

Propafenon-Hydrochlorid, Natriumalginat, mikrokristalline Cellulose (Typ PH 101) und Eudragit RS wurden in einem Vertikalmischer gemischt und mit 20 %iger Gelatinelösung granuliert. Die Abtrocknung des feuchten Granulates erfolgte in einem Wirbelschichttrockner mit 60°C warmer Zuluft. Nach dem Durchgeben durch ein Sieb geeigneter Maschenweite wurden Magnesiumstearat und mikrokristalline Cellulose (Typ PH 102) in einem Horizontalmischer zugemischt und anschließend die Mischung auf einer Rundläufer-Tablettenpresse zu Oblongtabletten verpreßt (Maße 18 x 8,7 mm). Der leicht lösliche Überzug wurde in einem Horizontalcoater aufgebracht.

Die In-vitro-Freisetzungsbestimmung in einer Paddle-Apparatur bei 50 rpm ergab folgende Werte (in %):

| | | |
|---|---|---|
| 1. | Stunde | 3,8 |
| 2. | Stunde | 5,5 |
| 3. | Stunde | 23,7 |
| 4. | Stunde | 43,0 |
| 6. | Stunde | 75,4 |
| 8. | Stunde | 89,5 |

Die In-vitro-Freisetzung der Retard-Bolusfilmtablette ist also ähnlich der der erfindungsgemäßen Retard-Mikrotabletten. Dennoch ist die In-vivo-Freisetzung völlig anders, und zwar erfindungsgemäß besser, vgl. Pharmaspiegel gem. Fig. 11.

**Patentansprüche**

**1.** Zylindrische Retardmikrotablette mit konvexer oder planer Ober- und Unterseite von β-Phenylpropiophenonderivaten der Formel I als Wirkstoff

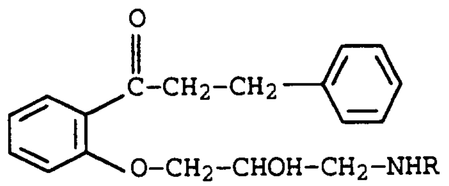

I

mit R= n-Propyl oder 1,1-Dimethylpropyl und deren pharmakologisch unbedenklichen Salzen, dadurch gekennzeichnet, daß

a) Höhe und Durchmesser unabhängig voneinander 1 bis 3 mm betragen,

b) der Wirkstoffgehalt im Bereich von 81 bis 99,9 Gew.-% der Mikrotablette liegt, jedoch ohne Berücksichtigung des Gewichts eines gegebenenfalls vorhandenen Überzugs,

c) die Wirkstoffdichte höher als 1 $g/cm^3$ ist,

d) im Paddle Modell nach USP bei 50 rpm nach 3 Stunden höchstens und nach 24 Stunden mindestens 80 % des Wirkstoffs freigesetzt sind,

e) die Freisetzungsgeschwindigkeit praktisch unabhängig vom Druck beim Pressen der Tabletten ist, und

f) die Tablette keinen retardierenden Hilfsstoff, aber 0,1 bis 5 Gew.-% eines Gleitmittels und 0 bis 18,9 Gew.-% anderer üblicher Hilfsstoffe enthält.

2. Tablette nach Anspruch 1, dadurch gekennzeichnet, daß sich in vivo ein ausgeprägtes Plasmaspiegelplateau mit einem PTF-Wert < 75 % ergibt und die Bioverfügbarkeit unabhängig von der eingenommenen Nahrung ist.

3. Tablette nach Anspruch 1 oder 2, dadurch gekennzeichnet, daß der Wirkstoff Propafenon-hydrochlorid ist.

4. Tablette nach einem der Ansprüche 1 bis 3, dadurch gekennzeichnet, daß Höhe und Durchmesser etwa gleich sind.

5. Gelatinekapsel, die 3 bis 200 Tabletten nach einem der Ansprüche 1 bis 4 mit gleicher oder unterschiedlicher Freisetzungsrate enthält.

6. Verfahren zur Herstellung von zylindrischen Retardmikrotabletten gemäß Anspruch 1, dadurch gekennzeichnet, daß man eine homogene Mischung von 81 bis 99,9 Gew.-% des granulierten Wirkstoffs mit einer Korngröße unter 1 mm, 0,1 bis 5 Gew.-% eines Gleitmittels und 0 bis 18,9 Gew.-% anderer üblicher, nicht retardierender Hilfsstoffe in eine zylindrische Form mit je 1 bis 3 mm Höhe und Durchmesser preßt und entformt.

## Claims

1. A cylindrical delayed release microtablet with a convex or flat upper side and lower side of β-phenylpropiophenone derivatives of the formula I as active ingredient

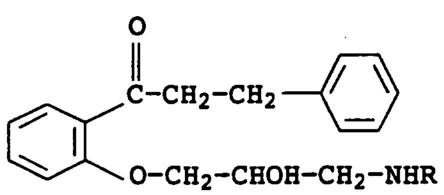

I

where R is n-propyl or 1,1-dimethylpropyl, and their pharmacologically acceptable salts, wherein

a) the height and diameter are, independently of one another, 1-3 mm,

b) the active ingredient content is in the range from 81 to 99.9% of the weight of the microtablet, but not taking into account the weight of any coating which is present,

c) the active ingredient density is greater than 1 g/cm$^3$,

d) the release of active ingredient in the USP paddle method at 50 rpm is 80% as a maximum after 3 hours and as a minimum after 24 hours,

e) the release rate is virtually independent of the pressure when compressing the tablets, and

f) the tablet contains no release-delaying ancillary substance but 0.1-5% by weight of a lubricant and 0-18.9% by weight of other conventional ancillary substances.

**2.** A tablet as claimed in claim 1, which in vivo results in a pronounced plasma level plateau with a PTF < 75% and whose bioavailability does not depend on the intake of food.

**3.** A tablet as claimed in claim 1 or 2, wherein the active ingredient is propafenone hydrochloride.

**4.** A tablet as claimed in any of claims 1 to 3, wherein the height and diameter are approximately the same.

**5.** A gelatin capsule which contains 3-200 tablets as claimed in any of claims 1 to 4 with identical or different release rates.

**6.** A process for producing cylindrical delayed release microtablets as claimed in claim 1, which comprises a homogeneous mixture of 81-99.9% by weight of the granulated active ingredient with a particle size below 1 mm, 0.1-5% by weight of a lubricant and 0-18.9% by weight of other conventional ancillary substances which do not delay release being compressed in a cylindrical mold with a height and diameter each of 1-3 mm and being removed from the mold.

## Revendications

**1.** Microcomprimés retard cylindriques à côté supérieur et à côté inférieur convexes ou plans de dérivés de la β-phénylpropiophénone de la formule I, à titre de principe actif

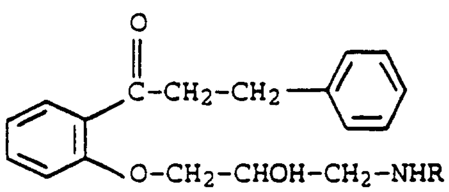

I

dans laquelle R représente le radical n-propyle ou le radical 1,1-diméthylpropyle et leurs sels pharmaceutiquement acceptables, caractérisés en ce que

a) hauteur et diamètre varient, indépendamment l'un de l'autre, de 1 à 3 mm,
b) la teneur en principe actif fluctue dans la plage de 81 à 99,9% en poids des microcomprimés compte non tenu cependant du poids d'un éventuel enrobage présent,
c) la masse volumique du principe actif est supérieure à 1 g/cm$^3$,
d) dans le Paddle Modell selon la USP (pharmacopée des Etats-Unis d'Amérique) à 50 tpm, après 3 heures au maximum et après 24 heures, au moins 80% du principe actif sont libérés,
e) la vitesse de libération est pratiquement indépendante de la pression exercée au cours de la compression des comprimés et
f) les comprimés ne contiennent pas d'adjuvant retardateur, mais cependant 0,1 à 5% en poids d'un lubrifiant et 0 à 18,9% en poids d'autres adjuvants usuels.

**2.** Comprimés suivant la revendication 1, caractérisés en ce que, in vivo, s'établit un plateau de niveau plasmatique

remarquable avec une valeur PTF inférieure à 75% et la biodisponibilité est indépendante de la nourriture ingérée.

**3.** Comprimés suivant la revendication 1 ou 2, caractérisés en ce que le principe actif est le chlorhydrate de propafénone.

**4.** Comprimés suivant l'une quelconque des revendications 1 à 3, caractérisés en ce que la hauteur et le diamètre sont à peu près identiques.

**5.** Gélules de gélatine qui contiennent 3 à 200 comprimés suivant l'une quelconque des revendications 1 à 4, à taux de libération identique ou différent.

**6.** Procédé de fabrication de microcomprimés retard cylindrique suivant la revendication 1, caractérisé en ce que l'on comprime un mélange homogène de 81 à 99,9% en poids du principe actif granulé, d'un calibre des grains inférieur à 1 mm, 0,1 à 5% en poids d'un lubrifiant et 0 à 18,9% en poids d'autres adjuvants non retardateurs, habituels, dans un moule cylindrique avec à chaque fois une hauteur et un diamètre de 1 à 3 mm et on les démoule.

FIG.1

Freisetzung Propafenon ret. Mikrotabl. gem. Bsp. 1
400 mg in Kapseln

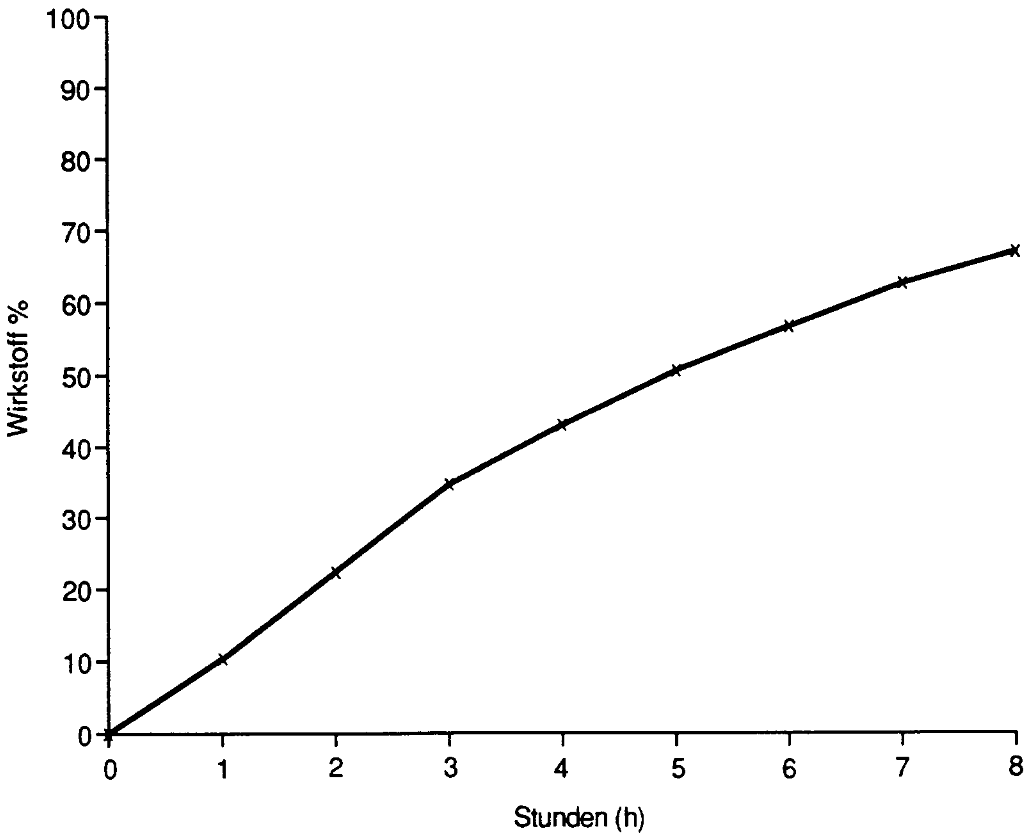

FIG.2

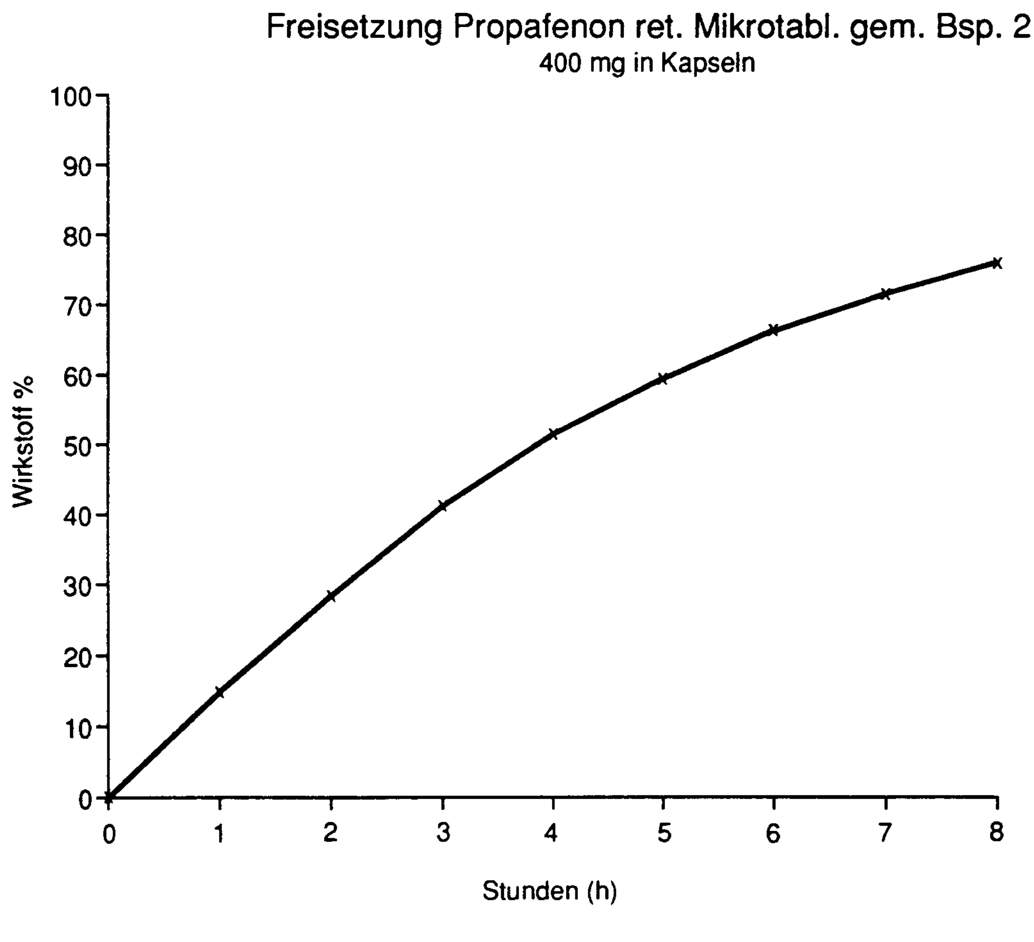
Freisetzung Propafenon ret. Mikrotabl. gem. Bsp. 2
400 mg in Kapseln
Wirkstoff %
100
90
80
70
60
50
40
30
20
10
0
0
1
2
3
4
5
6
7
8
Stunden (h)

FIG.3

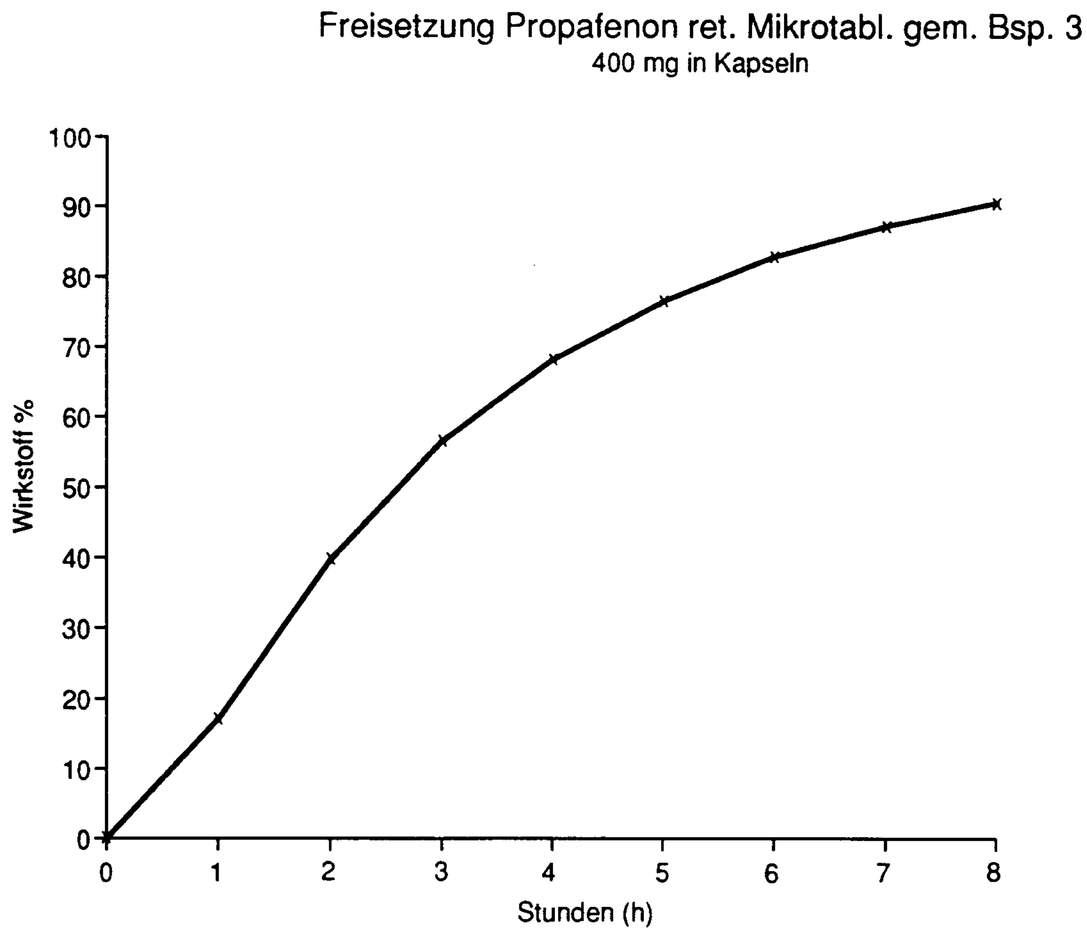
Freisetzung Propafenon ret. Mikrotabl. gem. Bsp. 3
400 mg in Kapseln
Wirkstoff %
0
10
20
30
40
50
60
70
80
90
100
0
1
2
3
4
5
6
7
8
Stunden (h)

FIG.4

Freisetzung Propafenon ret. Mikrotabl. gem. Bsp. 4
300 mg in Kapseln

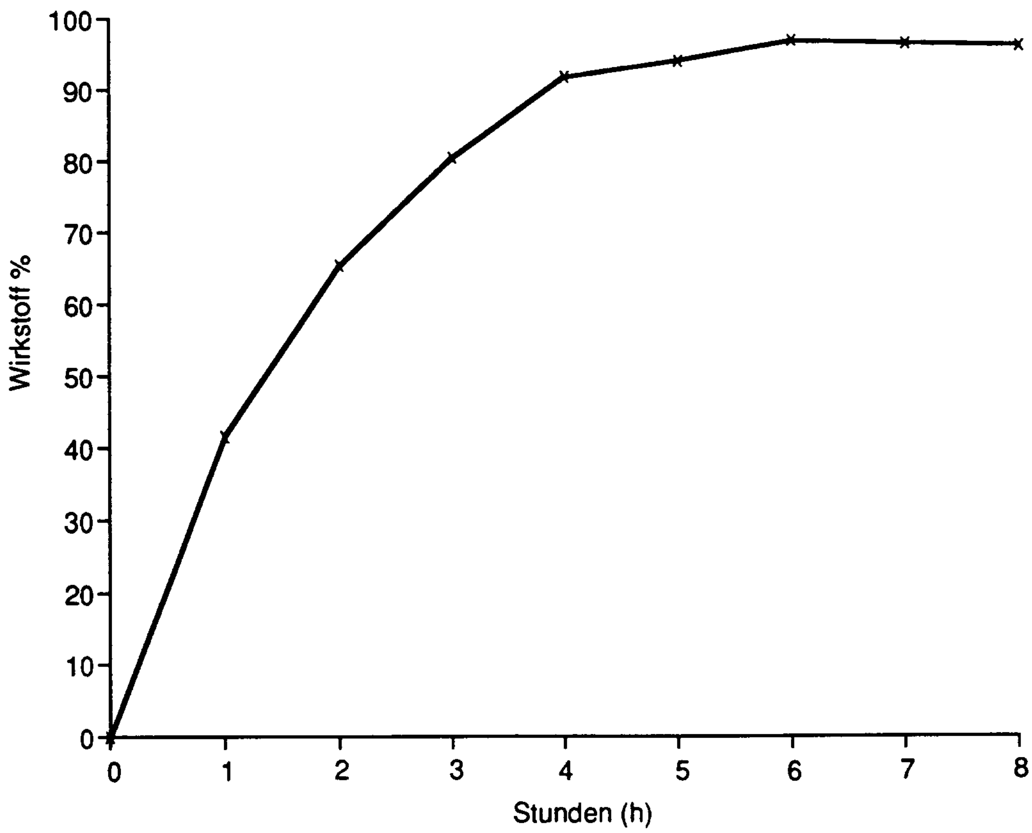

FIG.5

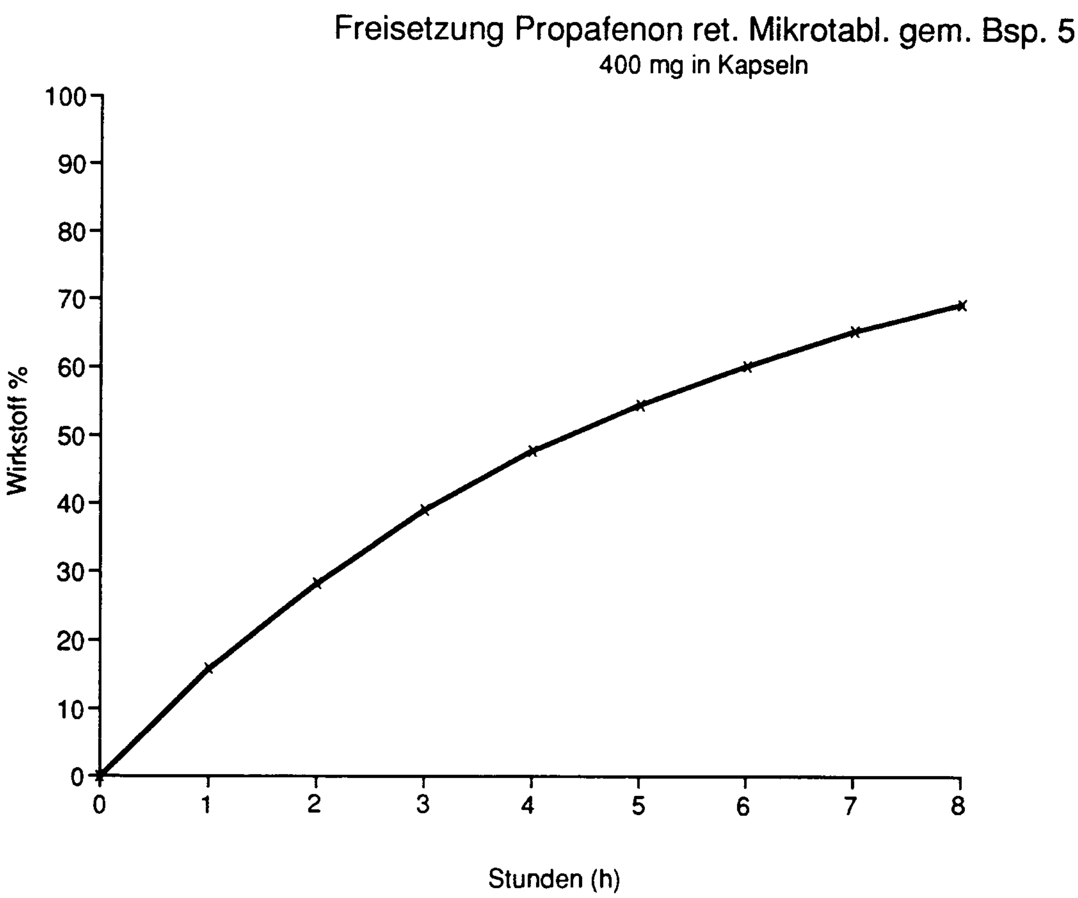
Freisetzung Propafenon ret. Mikrotabl. gem. Bsp. 5
400 mg in Kapseln
Wirkstoff %
0
10
20
30
40
50
60
70
80
90
100
0
1
2
3
4
5
6
7
8
Stunden (h)

FIG.6

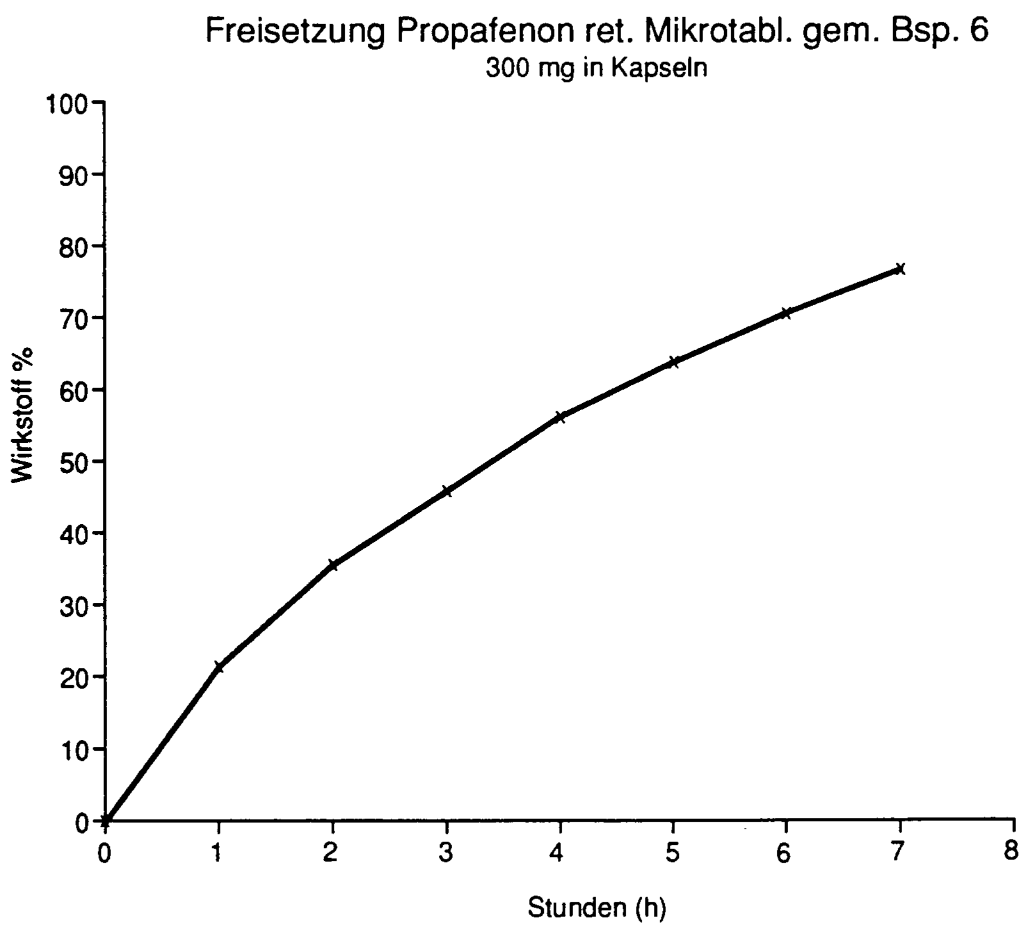
Freisetzung Propafenon ret. Mikrotabl. gem. Bsp. 6
300 mg in Kapseln
Wirkstoff %
100
90
80
70
60
50
40
30
20
10
0
0
1
2
3
4
5
6
7
8
Stunden (h)

FIG.7

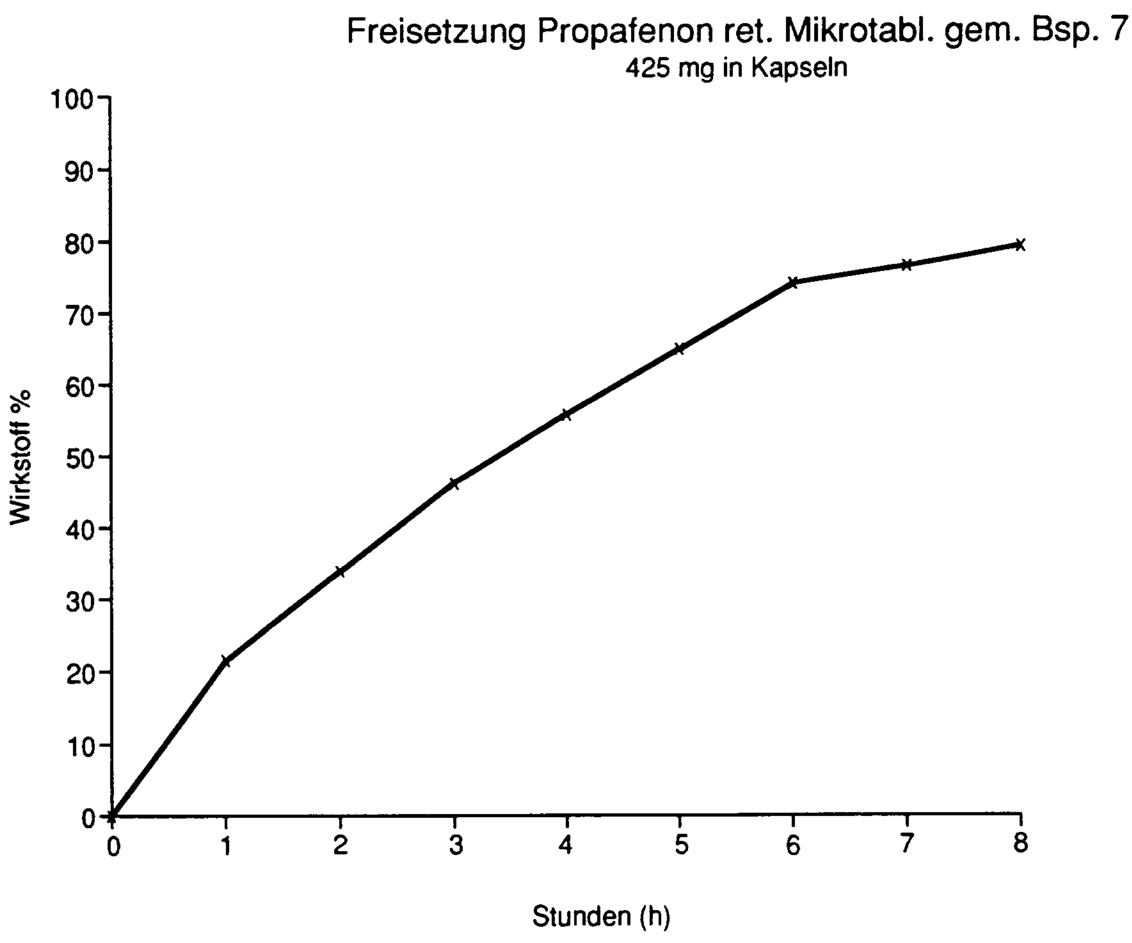
Freisetzung Propafenon ret. Mikrotabl. gem. Bsp. 7
425 mg in Kapseln
Wirkstoff %
100
90
80
70
60
50
40
30
20
10
0
0
1
2
3
4
5
6
7
8
Stunden (h)

FIG.8

Freisetzung Propafenon ret. Mikrotabl. gem. Bsp. 8
325 mg in Kapseln

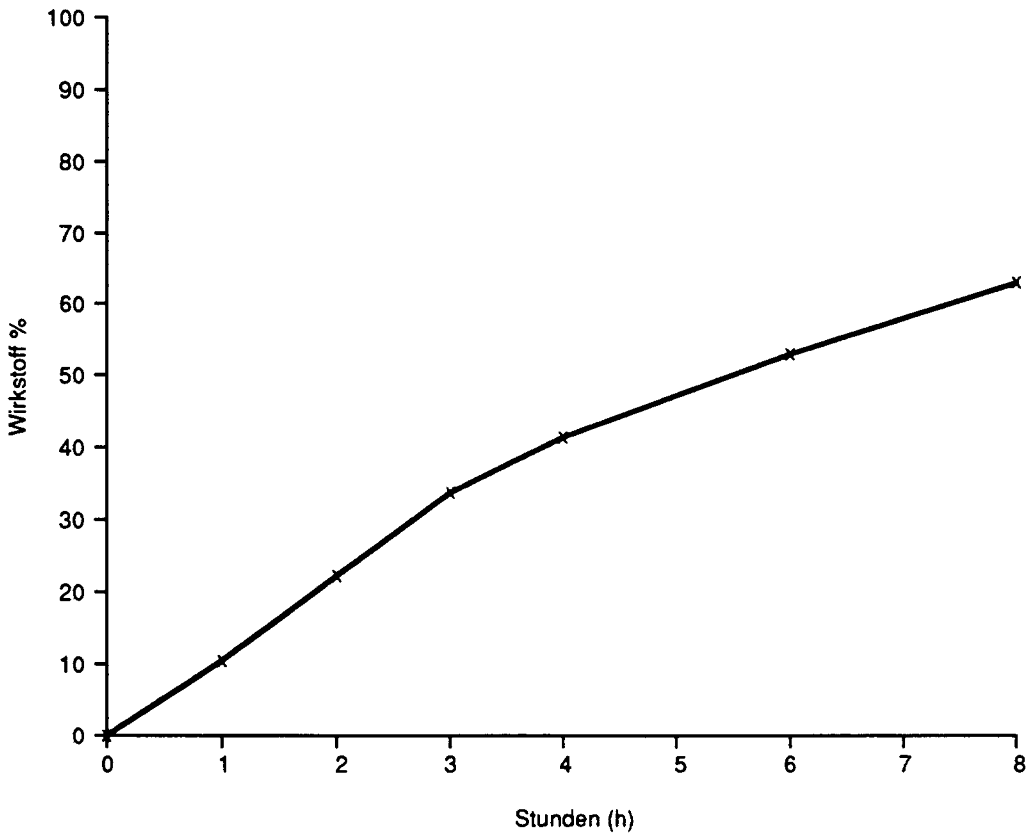

FIG.9

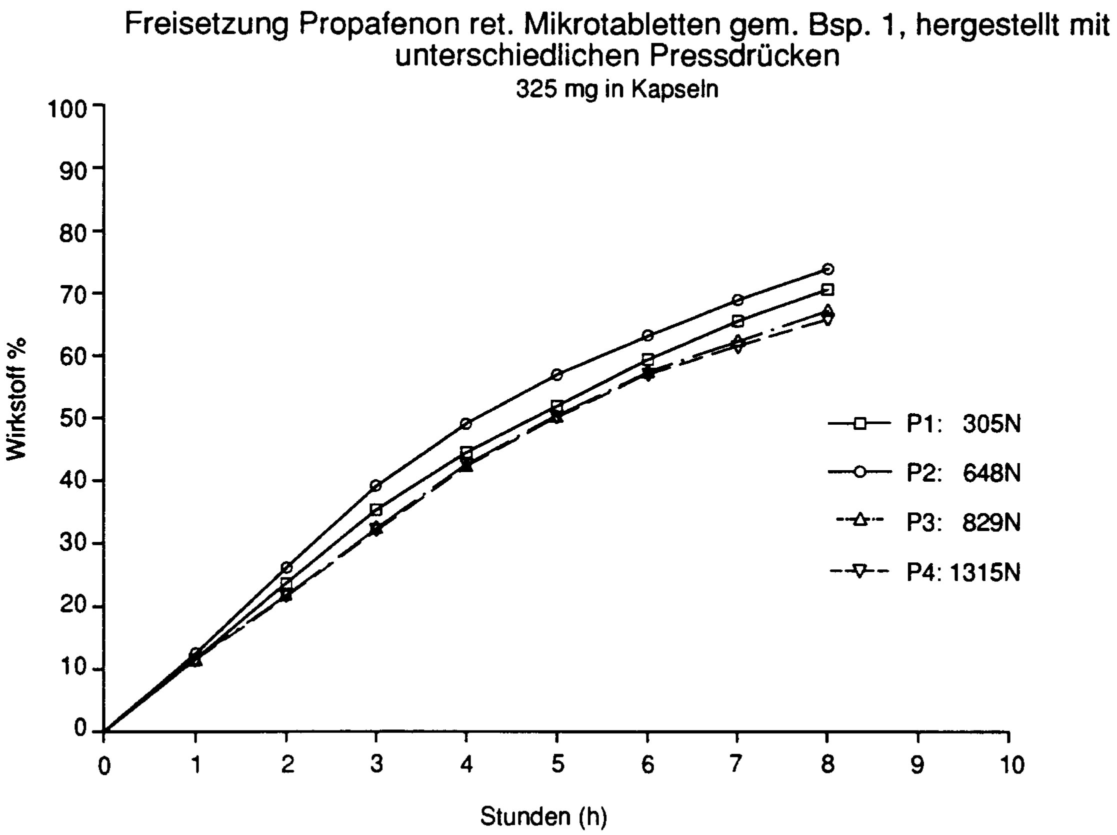
Freisetzung Propafenon ret. Mikrotabletten gem. Bsp. 1, hergestellt mit unterschiedlichen Pressdrücken
325 mg in Kapseln
Wirkstoff %
0
10
20
30
40
50
60
70
80
90
100
0
1
2
3
4
5
6
7
8
9
10
Stunden (h)
P1: 305N
P2: 648N
P3: 829N
P4: 1315N

FIG.10

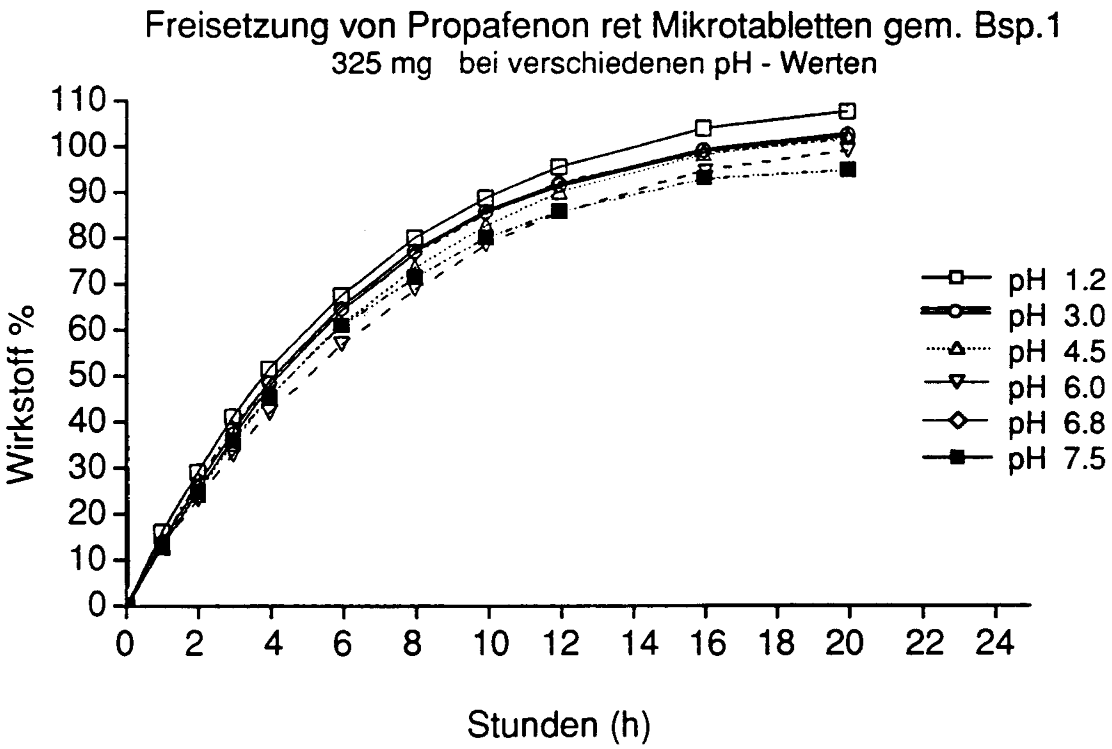
Freisetzung von Propafenon ret Mikrotabletten gem. Bsp.1
325 mg bei verschiedenen pH - Werten
110
100
90
80
70
60
50
40
30
20
10
0
Wirkstoff %
0 2 4 6 8 10 12 14 16 18 20 22 24
Stunden (h)
pH 1.2
pH 3.0
pH 4.5
pH 6.0
pH 6.8
pH 7.5

FIG.11

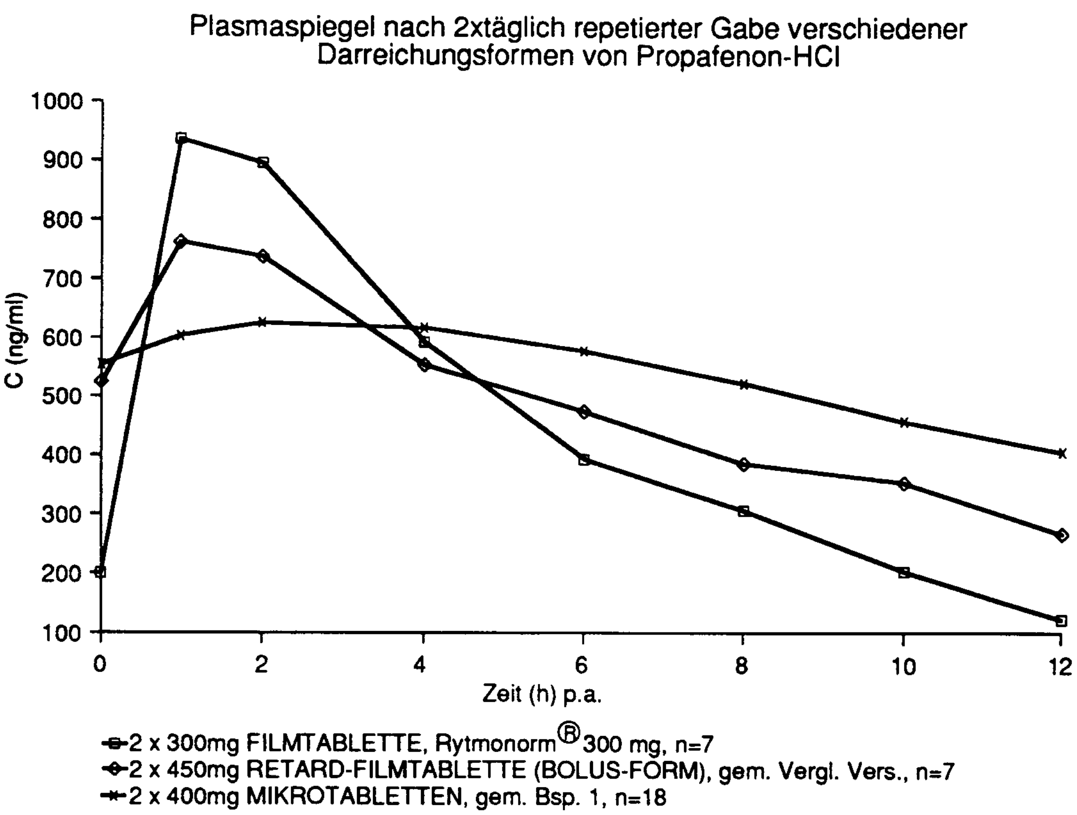
Plasmaspiegel nach 2xtäglich repetierter Gabe verschiedener Darreichungsformen von Propafenon-HCl
C (ng/ml)
1000
900
800
700
600
500
400
300
200
100
0
2
4
6
8
10
12
Zeit (h) p.a.
2 x 300mg FILMTABLETTE, Rytmonorm® 300 mg, n=7
2 x 450mg RETARD-FILMTABLETTE (BOLUS-FORM), gem. Vergl. Vers., n=7
2 x 400mg MIKROTABLETTEN, gem. Bsp. 1, n=18